# FASCICULE DE BREVET EUROPEEN

(11) **EP 3 329 255 B1**
(45) Date de publication et mention de la délivrance du brevet: **25.11.2020**
(21) Numéro de dépôt: 16744775.4
(22) Date de dépôt: 28.07.2016
(51) Int. Cl.: G01N 21/64, G01N 21/63, G01N 33/58

(54) **PROCEDE DE DETECTION D'ESPECES OXYDANTES**
VERFAHREN ZUR ERKENNUNG VON OXIDIERENDEN SPEZIES
METHOD FOR DETECTING OXIDISING SPECIES

(30) Priorité: 29.07.2015 FR 1557275
(43) Date de publication de la demande: 06.06.2018
(73) Titulaire: Ecole Polytechnique, 91128 Palaiseau Cedex (FR); Centre National de la Recherche Scientifique, 75794 Paris Cedex 16 (FR)
(72) Inventeur: BOUZIGUES, Cédric, 75016 Paris (FR); ABDESSELEM, Mouna, 3000 Leuven (BE); ALEXANDROU, Antigoni, 91120 Palaiseau (FR); GACOIN, Thierry, 91440 Bures sur Yvette (FR); BOILOT, Jean-Pierre, 92190 Meudon (FR)
(74) Mandataire: Nony
(86) Numéro de dépôt international: PCT/EP2016/068096
(87) Numéro de publication internationale: WO 2017/017233

(56) Documents cités:
- CÉDRIC BOUZIGUES ET AL: "Cartographier la concentration intracellulaire d'espèces oxygénées réactives", M/S MEDECINE SCIENCES., vol. 30, no. 10, 1 octobre 2014 (2014-10-01), pages 848-850, XP055275410, FR ISSN: 0767-0974, DOI: 10.1051/medsci/20143010010
- DIDIER CASANOVA ET AL: "Single europium-doped nanoparticles measure temporal pattern of reactive oxygen species production inside cells", NATURE NANOTECHNOLOGY, vol. 4, no. 9, 1 septembre 2009 (2009-09-01), pages 581-585, XP055027045, ISSN: 1748-3387, DOI: 10.1038/nnano.2009.200 cité dans la demande
- MOUNA ABDESSELEM ET AL: "Multifunctional Rare-Earth Vanadate Nanoparticles: Luminescent Labels, Oxidant Sensors, and MRI Contrast Agents", ACS NANO, vol. 8, no. 11, 25 novembre 2014 (2014-11-25), pages 11126-11137, XP055275081, US ISSN: 1936-0851, DOI: 10.1021/nn504170x
- Alok d. Wessel ET AL: "The Mechanical Properties of Early Drosophila Embryos Measured by High-Speed Video Microrheology", BIOPHYSICAL JOURNAL, vol. 108, no. 8, 1 April 2015 (2015-04-01) , pages 1899-1907, XP055536186, AMSTERDAM, NL ISSN: 0006-3495, DOI: 10.1016/j.bpj.2015.02.032
- MAHOU PIERRE ET AL: "Multiphoton light-sheet microscopy using wavelength mixing: fast multicolor imaging of the beating Zebrafish heart with low photobleaching", PROGRESS IN BIOMEDICAL OPTICS AND IMAGING, SPIE - INTERNATIONAL SOCIETY FOR OPTICAL ENGINEERING, BELLINGHAM, WA, US, vol. 9329, 3 March 2015 (2015-03-03), pages 93290Z-93290Z, XP060049197, ISSN: 1605-7422, DOI: 10.1117/12.2079176 ISBN: 978-1-5106-0027-0

## Description

L'invention se rapporte à un procédé utile pour l'analyse d'espèces oxydantes et à un système de détection d'espèces oxydantes, adapté à mettre en œuvre ce procédé.

De nombreux procédés ont déjà été proposés visant à détecter la présence d'espèces oxydantes dans un échantillon.

Les espèces oxydantes plus particulièrement ciblées sont notamment des messagers cellulaires impliquées dans diverses voies de signalisation. Leur régulation est primordiale dans les réactions immunitaires et dans des pathologies telles que certains cancers, maladies cardiovasculaires ou maladies neurodégénératives. Il est donc particulièrement intéressant de disposer de méthodes efficaces de détermination de la présence, de quantification de cette présence et de l'évolution temporelle de la concentration d'espèces oxydantes dans un échantillon à tester, notamment dans un échantillon biologique.

Il est à noter cependant que la détection d'espèces oxydantes présente de nombreuses autres applications. La demande FR-A-2 980 847 présente ainsi, à titre d'exemple, une méthode de détection d'explosifs basée sur la détection d'espèces oxydantes.

Certains des procédés connus proposent d'utiliser un agent fluorescent organique (ou fluorophore) dont la luminescence est exaltée par la présence d'espèces oxydantes dans l'échantillon. La DiChloroFluorescéine est un exemple d'un tel agent fluorescent organique, dont la fluorescence est exaltée en présence d'oxydant, pouvant être utilisé en milieu vivant.

Cependant, il est connu qu'un tel agent fluorescent organique subit un fort photoblanchiment et est susceptible de s'oxyder spontanément en milieu ambiant, ce qui ne permet pas de réaliser, en pratique, des mesures quantitatives de longue durée. En outre, l'augmentation de fluorescence fait suite à une réaction d'oxydation irréversible, ce qui ne permet pas de réaliser des mesures résolues en temps.

Il est également connu de mettre en œuvre une protéine fluorescente en tant qu'agent fluorescent, notamment dans un organisme génétiquement modifié. cpYFP, HyPer (1,2 et 3) ou roGFP sont des exemples connus d'une telle protéine fluorescente. Cependant, ces agents peuvent être spécifiques à certaines espèces oxydantes seulement, présenter un temps de réponse long ou avoir une gamme de détection restreinte, avec par exemple une saturation à 500 nmol.L⁻¹ pour la plus performante des protéines Hyper, Hyper3, incompatible avec certaines applications, notamment les applications biologiques. Bilan et al., ACS Chem. Biol 2013 ont cependant montré que ces protéines fluorescentes pouvaient être utilisées pour la détection d'espèces oxydantes dans des cellules vivantes, mais sans obtenir de mesure de la concentration absolue d'oxydants.

Par ailleurs, il est connu de l'article « Single europium-doped nanoparticles measure temportal pattern of reactive oxygen species production inside cells », de Didier Casanova et al., Nature Nanotechnology, vol. 4, Septembre 2009, un procédé de détection quantitative d'espèces oxydantes dans lequel des nanoparticules d'Y_{0,6}Eu_{0,4}VO₄ dopées à l'europium à l'état d'oxydation III sont utilisées comme agent photoluminescent. L'utilisation de ces particules requiert donc au préalable leur réduction pour les rendre oxydables. Cette réduction y est réalisée *in situ* sous irradiation laser de manière à disposer d'élément europium au degré d'oxydation II et donc sous une forme oxydable par une ou plusieurs espèces oxydantes. Or, cette photo-réduction dédiée à générer l'espèce europium oxydable s'avère parfois délétère à l'égard des cellules conjointement présentes. Cette photo-réduction est de plus difficilement praticable sur des échantillons macroscopiques ou *in vivo.*

En outre, pour déterminer l'évolution temporelle de la concentration en espèces oxydantes dans l'échantillon testé, le procédé décrit dans cet article nécessite de déterminer l'intensité lumineuse des nanoparticules et la dérivée par rapport au temps de ce signal. La détermination de cette dérivée nécessite plusieurs mesures successives de l'intensité lumineuse, ce qui limite la résolution temporelle d'un tel procédé à environ 30 s.

Enfin, un tel procédé s'appuie sur la mesure d'une intensité absolue de luminescence et suppose donc l'observation des nanoparticules sous une forme individualisée, ce qui empêche toute détection dans un volume macroscopique.

La publication Abdesselem et al. (« Multifunctional Rare-Earth Vanadate Nanoparticles : Luminescent Labels, Oxidant Sensors, and MRI Contrast Agents », ACS NANO, vol. 8, no. 11, 25 novembre 2014, pages 11126-11137) décrit la mise en œuvre, à titre d'alternative aux nanoparticules YVO₄ :Eu mises en œuvre selon l'article précité de Casanova *et al.,* de nanoparticules de de GdVO₄ :Eu, pour la détection de H₂O₂ intracellulaire.

Le but de la présente invention est de proposer une méthode de détection d'espèces oxydantes ne présentant pas les inconvénients des procédés connus de l'art antérieur. Notamment, l'invention vise à proposer une méthode qui soit compatible avec une mesure volumique et/ou présente une meilleure résolution temporelle et qui selon une variante préférée ne requiert pas de générer *in situ* une espèce oxydable.

À cette fin, l'invention propose à titre principal un procédé utile pour l'analyse d'espèces oxydantes dans un échantillon, en particulier dans un échantillon biologique, comprenant les étapes consistant à :
i) mettre en contact l'échantillon avec un premier agent photoluminescent, et un deuxième agent optiquement actif, au moins le premier agent photoluminescent comportant des nanoparticules dopées aux terres rares et oxydables, avec
   - la luminescence du premier agent photoluminescent variant, à au moins une première longueur d'onde, avec la quantité d'espèces oxydantes, et
   - le signal émis par le deuxième agent optiquement actif étant, à au moins une seconde longueur d'onde distincte de la première longueur d'onde, constant avec la quantité d'espèces oxydantes, ou
   variant avec la quantité d'espèces oxydantes dans un sens opposé à la luminescence du premier agent photoluminescent,
ii) exciter le premier agent photoluminescent et le deuxième agent optiquement actif dans l'échantillon ;
iii) mesurer l'intensité lumineuse de l'échantillon à au moins ladite première longueur d'onde et au moins ladite seconde longueur d'onde, et
iv) apprécier la présence et/ou quantité d'espèce(s) oxydante(s) par interprétation desdites intensités lumineuses mesurées, et le cas échéant par référence à des valeurs étalon.

Au sens de l'invention, l'expression « analyse d'espèces oxydantes » couvre l'aspect détection ou encore caractérisation qualitative de la présence ou non d'espèces oxydantes et également l'aspect dosage ou encore caractérisation quantitative des espèces oxydantes.

Au sens de l'invention, on entend par agent optiquement actif, un agent adapté à émettre des photons suite à une excitation par des photons, éventuellement de longueurs d'ondes différentes.

Ainsi, le procédé proposé repose sur la mesure simultanée de l'intensité d'émission d'un premier agent photoluminescent et d'un deuxième agent optiquement actif, l'intensité d'émission d'au moins l'un des deux agents variant en fonction de la présence d'espèces oxydantes. La double mesure simultanée est possible en choisissant des agents ayant des longueurs d'ondes d'émission distinctes. Les inventeurs ont démontré qu'en outre cette double mesure permet de réaliser des mesures en volume sur un échantillon macroscopique, ou microscopique, avec une meilleure résolution temporelle, comme cela sera expliqué plus en détails par la suite.

Plus précisément, la première longueur d'onde, correspond à une longueur d'onde d'émission représentative de la forme oxydée ou de la forme réduite du premier agent photoluminescent.

Pour sa part, la seconde longueur d'onde, correspond à une longueur d'onde d'émission spécifique du deuxième agent optiquement actif et le cas échéant représentative de sa forme réduite ou oxydée.

Selon une variante préférée, la première longueur d'onde de luminescence est représentative d'une forme oxydée du premier agent photoluminescent. En conséquence, la présence d'espèces oxydantes sera sanctionnée par la visualisation d'une intensité lumineuse accrue à cette longueur d'onde.

Selon une première variante, le premier agent luminescent est mis en œuvre en combinaison avec un agent optiquement actif dont l'intensité lumineuse ne varie pas avec la concentration en espèces oxydantes. Dans cette alternative, l'intensité lumineuse spécifique à l'agent optiquement actif peut être directement utilisée à titre de valeur étalon pour sanctionner un accroissement ou non de l'intensité lumineuse spécifique au premier agent luminescent, un accroissement étant révélateur de la présence d'au moins une espèce oxydante.

Ce mode de réalisation reposant sur l'association du premier agent luminescent avec un second agent optiquement actif est particulièrement avantageux pour une analyse dans un volume macroscopique et notamment dans un environnement non surfacique, par exemple un volume liquide ou dans une épaisseur d'échantillon.

Selon une seconde variante, le premier agent luminescent est mis en œuvre en combinaison avec un agent optiquement actif dont l'intensité lumineuse de ladite seconde longueur d'onde attitrée varie également en fonction de la concentration d'espèces oxydantes mais avec une évolution opposée à celle manifestée par le premier agent luminescent. Les nanoparticules des deux types sont observées individuellement, par exemple après dépôt sur une surface ou internalisation dans une cellule. Ainsi dans la variante préférée du procédé selon l'invention où la première longueur d'onde de luminescence est représentative d'une forme oxydée du premier agent photoluminescent, la deuxième longueur d'onde spécifique à l'agent optiquement actif est représentative de la forme réduite de cet agent. Ainsi, la présence d'espèces oxydantes sera sanctionnée par la visualisation d'une diminution de l'intensité lumineuse de cette seconde longueur d'onde.

Dans cette alternative, l'interprétation des mesures des deux intensités lumineuses est avantageusement réalisée en se reportant à des valeurs étalons préétablies.

Plus précisément, la quantité d'espèces oxydantes dans l'échantillon peut être déterminée par lecture sur une nappe étalon de la valeur correspondant au couple d'intensités lumineuses mesurées à l'étape iii). Cette nappe étalon est une nappe préétablie au moyen de mesures réalisées avec des échantillons à quantité connue en espèces oxydantes, de préférence dans des conditions identiques à celles de l'étude de l'échantillon, ces conditions identiques incluant notamment l'un au moins parmi le solvant, le pH et la température de test.

Ce mode de réalisation reposant sur l'association du premier agent luminescent avec un second agent optiquement actif à intensité lumineuse variable est particulièrement avantageux dans la mesure où il permet d'accéder à partir d'une unique mesure du couple d'intensité lumineuse représentatif des premier et deuxième agents, et donc de manière rapide, limitée uniquement par la cadence d'acquisition du dispositif d'enregistrement, et qui plus est fiable, à une valeur quantitative de la concentration en espèces oxydantes dans l'échantillon à analyser.

Selon une variante de réalisation, le procédé selon l'invention met en œuvre à titre de premier agent photoluminescent des nanoparticules d'AₓEu₁₋ₓ(VO₄)_{y}(PO₄)_{(1-y)}, en particulier d'AₓEu₁₋ₓVO₄, où A est l'un parmi Y, Gd et La, 0 ≤ x ≤ 1 et 0 ≤ y ≤ 1 dans lesquelles l'élément europium est au moins partiellement à un état réduit et donc de degré d'oxydation II.

Compte-tenu de la présence d'élément europium à l'état d'oxydation réduit, les nanoparticules correspondantes sont avantageusement directement oxydables par opposition à des nanoparticules d'AₓEu₁₋ₓ(VO₄)_{y}(PO₄)_{(1-y)}, dites oxydes, car contenant l'élément Eu uniquement à un état d'oxydation III.

La demande décrit également un procédé utile pour l'analyse d'espèces oxydantes dans un échantillon, en particulier dans un échantillon biologique, comprenant les étapes consistant à :
i) disposer de nanoparticules photoluminescentes et oxydables d'AₓEu₁₋ₓ(VO₄)_{y}(PO₄)_{(1-y)}, où A est l'un parmi Y, Gd et La, 0 ≤ x ≤ 1 et 0 ≤ y ≤ 1 et contenant de l'élément europium à un degré d'oxydation II,
ii) introduire lesdites nanoparticules dans l'échantillon à doser,
iii) exciter lesdites nanoparticules,
iv) mesurer l'intensité lumineuse émise par l'échantillon à au moins une longueur d'onde représentative de la forme oxydée ou de la forme réduite desdites nanoparticules, et
iv) apprécier la présence et/ou quantité d'espèce(s) oxydante(s) par interprétation de ladite mesure, le cas échéant par référence à un étalon ou étalonnage.

Un procédé selon l'invention est particulièrement utile pour l'analyse notamment la détection et/ou le dosage d'espèces oxydantes dans un échantillon.

Ainsi, un procédé selon l'invention peut être mis en œuvre pour étudier l'impact écologique des rejets d'oxydants issus des procédés industriels de nettoyage et de blanchiment. Il peut aussi permettre la détection d'espèces oxydantes en phase vapeur pour la détection d'explosifs.

Néanmoins, un procédé selon l'invention est tout particulièrement intéressant pour la caractérisation des espèces oxydantes réactives, dites ROS, relevant du domaine du vivant.

Ainsi, la demande décrit l'utilisation d'un procédé selon l'invention à des fins de diagnostic, notamment de troubles physiologiques, pathologiques ou non, associés à une expression non physiologiquement acceptable d'une ou plusieurs espèces oxydantes réactives.

Selon un autre des aspects, la présente invention vise ainsi l'utilisation d'un procédé selon l'invention à des fins de diagnostic *ex-vivo* ou *in vitro,* notamment de troubles physiologiques, pathologiques ou non, associés à une expression non physiologiquement acceptable d'une ou plusieurs espèces oxydantes réactives.

En effet, les espèces oxydantes réactives sont essentielles et impliquées dans un grand nombre de fonctions biologiques, principalement la transduction du signal, la neurotransmission, la relaxation des muscles lisses, l'agrégation plaquettaire, la modulation de la pression artérielle, le contrôle du système immunitaire, la régulation de la croissance cellulaire, la synthèse de nombreuses molécules biologiques, l'inflammation et le métabolisme de xénobiotiques.

À titre représentatif des espèces oxydantes réactives susceptibles d'être analysées dans un échantillon biologique peuvent notamment être cités les radicaux libres tels que les radicaux superoxyde (O₂·⁻), hydroperoxyde (HO₂·⁻), hydroxyle (HO·), peroxyde (ROO·), monoxyde d'azote (NO·), dioxyde d'azote (NO₂·) mais également les espèces non radicalaires comme le peroxyde d'hydrogène (H₂O₂), l'oxygène singulet (¹O₂), l'acide hypochloreux (HOCl), l'anion peroxynitrite (ONOO⁻), l'acide peroxynitreux (ONOOH), l'anion nitrosoperoxycarbonate (ONOOCO₂⁻), le cation nitronium (NO₂⁺) et le trioxyde d'azote (N₂O₃) par exemple.

En conséquence, lorsque ces espèces oxydantes sont produites en excès ou à l'inverse lorsque leur teneur physiologique s'avère insuffisante, il en découle des troubles physiologiques à l'image de l'oxydation de molécules biologiques telles que certaines protéines par exemple. De même la caractérisation d'une quantité non conforme aux quantités physiologiques usuelles peut être un indice de la manifestation d'un dysfonctionnement de métabolisme ou d'un état pathologique de type inflammation par exemple.

Ainsi, un procédé selon l'invention peut permettre de doser efficacement la quantité d'une ou plusieurs espèces oxydantes dans un échantillon biologique et de sanctionner à partir de la mesure effectuée si la concentration de ces espèces est ou non physiologiquement acceptable.

Un échantillon biologique, peut notamment être par exemple un organisme vivant, ou de tissus biologiques, notamment isolés de tout organisme vivant (tissus *ex vivo* ou fixés), des cellules, ou encore une solution contenant notamment des molécules biologiques.

Le fait que le procédé soit efficace à une échelle volumique, rend possible sa mise en œuvre au niveau d'une cible biologique *in vivo* ou d'un extrait de tissus ou de liquide biologique.

Ainsi, le procédé selon l'invention est avantageusement réalisé sur un échantillon volumique et est compatible avec une utilisation *in vivo, ex-vivo* et *in vitro.* À titre d'exemple d'utilisation, il est possible de réaliser une mesure utilisant une cuve contenant l'échantillon à analyser. Les deux agents y sont introduits en solution, excités par des sources spécifiques et l'intensité lumineuse associée à chacun est collectée sur deux photomultiplicateurs. L'analyse d'un tissu peut quant à elle être réalisée par injection du mélange de nanoparticules et détection avec séparation spectrale sur un macroscope.

Selon une variante de réalisation particulière, le procédé selon l'invention est mis en œuvre pour une utilisation *ex-vivo* à des fins de diagnostic, notamment de troubles physiologiques, pathologiques ou non, associés à une expression non physiologiquement acceptable d'une ou plusieurs espèces oxydantes réactives.

Selon encore un autre de ses aspects, la présente invention vise l'utilisation, *ex-vivo* ou in vitro , d'un procédé selon l'invention à des fins de criblage de l'efficacité d'un actif à l'égard d'un trouble, pathologique ou non, et associé à une surexpression d'une ou plusieurs espèces oxydantes réactives (ROS).

Ainsi, un procédé selon l'invention peut être mis en œuvre pour analyser la quantité en espèces oxydantes dans un échantillon biologique issu d'un patient traité avec un actif thérapeutique et apprécier la valeur de la mesure ainsi obtenue par comparaison à une valeur de référence représentative par exemple d'un échantillon biologique du même patient mais avant traitement.

Ce type de criblage peut également être réalisé à l'échelle expérimentale pour tester en laboratoire un panel d'actifs potentiels et dont l'efficacité peut être sanctionnée via le dosage d'espèces oxydantes, évaluées en leur présence et absence.

La demande décrit également un système pour mettre en œuvre un procédé selon l'invention comprenant :
- un dispositif d'illumination laser à une ou deux longueurs d'onde pour exciter le premier agent photoluminescent et le deuxième agent optiquement actif dans l'échantillon,
- un dédoubleur spectral pour filtrer les émissions du premier agent photoluminescent et du deuxième agent optiquement actif selon des longueurs d'ondes distinctes et ainsi former deux images filtrées distinctes de l'échantillon, et
- des moyens de détermination de l'intensité lumineuse d'au moins des points des deux images filtrées.

L'invention sera mieux comprise à la lecture de la description qui va suivre, donnée uniquement à but illustratif, et non limitatif, en regard des dessins annexés parmi lesquels :
- la figure 1 représente un schéma fonctionnel d'un procédé de détection d'espèces oxydantes ;
- la figure 2 représente le niveau de l'intensité lumineuse de la luminescence de nanoparticules de Gd_{0,6}Eu_{0,4}VO₄ après traitement par une solution de NaBH₄ (1 mol.l⁻¹) ;
- la figure 3 représente des courbes de réponse en intensité de luminescence de particules de Gd_{0,6}Eu_{0,4}VO₄ réduites et de YAG:Ce, respectivement, dans des milieux oxydants de concentration connue en peroxyde d'hydrogène ;
- la figure 4 représente un exemple de nappe indiquant une concentration en peroxyde d'hydrogène dans un échantillon en fonction d'un couple d'intensités lumineuses mesurées ;
- la figure 5 représente schématiquement un système permettant la mise en œuvre du procédé de la figure 1 ;
- la figure 6 représente un exemple d'observation d'un échantillon, selon l'exemple 1, de cellules musculaires lisses vasculaires de souris en culture, sous lumière blanche ;
- la figure 7 représente un exemple d'observation du même échantillon, selon l'exemple 1, dans lequel les nanoparticules indiquées sur la figure ont été internalisées dont la luminescence est détectée aux longueurs d'onde 550 nm et 617 nm ;
- la figure 8 représente l'évolution dans le temps de l'intensité lumineuse d'agents photoluminescents en contact avec l'échantillon de la figure 6 après injection d'endothéline, selon l'exemple 1 ;
- la figure 9 représente la concentration en peroxyde d'hydrogène dans l'échantillon de la figure 6 obtenu selon l'exemple 1, après injection d'endothéline, déterminée à partir de la nappe présentée figure 4 ;
- la figure 10 représente l'évolution de luminescence d'une solution dans une cuve, selon l'exemple 2, contenant un mélange de particules YAG:Ce et GdVO₄:Eu après ajout de 2 mM de peroxyde d'hydrogène (à t=0), détectée à 550 nm et 617 nm sur deux photomultiplicateurs ;
- la figure 11 représente les clichés obtenus par microscopie de fluorescence et dédoublement spectral d'un échantillon selon l'exemple 3 de cellules musculaires lisses vasculaires de souris en culture ;
- la figure 12 représente l'évolution, selon l'exemple 3, des photoluminescences normalisées par leur valeur initiale des nanoparticules YAG :Ce (au-dessous de 1) et GdVO₄ :Eu (au-dessus de 1) obtenues après stimulation par ET-1, avec ou sans inhibition des EGFRs ; et
- la figure 13 représente l'évolution temporelle des concentrations absolues en peroxyde d'hydrogène dans les deux conditions cellulaire (normales ou EGFR inhibés) déterminées selon l'exemple 3.

### DESCRIPTION DETAILLEE

Le procédé de détection d'espèces oxydantes 10 comprend une première étape 12 de mise en contact de l'échantillon avec un premier agent photoluminescent et un deuxième agent optiquement actif.

Comme indiqué précédemment, par agent optiquement actif, on entend un agent adapté à émettre des photons suite à une excitation par des photons, éventuellement de longueurs d'ondes différentes. Ainsi, un agent photoluminescent est un tel agent optiquement actif. Un agent de génération de seconde harmonique est également un agent optiquement actif au sens de la présente demande. Un exemple d'un tel agent de génération de seconde harmonique est par exemple des particules de KTP - pour titanyl phosphate de potassium (KTiOPO₄). Dans la suite de la description, le deuxième agent optiquement actif est un deuxième agent photoluminescent. Sauf mention contraire, ce qui est décrit pour le deuxième agent, photoluminescent, vaut également dans le cas où ce deuxième agent est optiquement actif, notamment un agent de génération de seconde harmonique.

Le premier agent photoluminescent comporte des nanoparticules dopées aux terres rares.

Par nanoparticules, on entend ici des particules dans le diamètre est de l'ordre du nanomètre, notamment supérieur à 1 nm et/ou inférieur à 500 nm. Par « diamètre », on entend ici la plus grande dimension de la nanoparticule.

Dans un mode de réalisation préféré, le deuxième agent, photoluminescent, comporte également des nanoparticules dopées aux terres rares.

Les premier et deuxième agents photoluminescents présentent des longueurs d'ondes de luminescence distinctes. En d'autres termes, il existe au moins une première longueur d'onde à laquelle seule la lumière émise par le premier agent photoluminescent est détectable et au moins une deuxième longueur d'onde, distincte de la première, à laquelle seule la lumière émise par le deuxième agent peut être détectée. On choisit de préférence les agents photoluminescents de telle sorte que leurs largeurs de raies soient suffisamment faibles pour être séparées spectralement. Ceci permet une détection plus aisée des émissions des premier et deuxième agents photoluminescents. Pour faciliter encore cette détection et pour une plus grande précision, on choisit des agents photoluminescents dont les longueurs d'ondes d'émissions maximales sont distinctes et on réalise la détection à ces longueurs d'ondes d'émission maximales.

En outre, le premier agent photoluminescent est choisi tel que l'intensité de sa luminescence émise à la longueur d'onde détectée (en abrégé ci-après, sa luminescence) varie avec la quantité d'espèces oxydantes en présence de laquelle il se trouve. Dans la suite, on considère le cas où l'intensité lumineuse de la luminescence du premier agent photoluminescent est croissante avec la quantité d'espèces oxydantes en présence desquelles il se trouve.

Les agents photoluminescents peuvent être déposés de façon à obtenir une faible densité surfacique sur ou dans un échantillon, lequel peut par exemple être reçu sur une lamelle de verre. Par faible densité surfacique, on entend une densité surfacique inférieure à 1 µm⁻².

Toutefois, le ou les agents photoluminescents considérés dans les procédés selon l'invention peuvent également être avantageusement directement introduits dans un échantillon volumique par exemple un volume d'échantillon liquide ou dans la masse d'un échantillon.

### Premier agent photoluminescent

Le premier agent photoluminescent comprend avantageusement des nanoparticules d'AₓEu₁₋ₓ(VO₄)_{y}(PO₄)_{(1-y)}, où A est l'un parmi Y et Gd et La, 0 ≤ x ≤ 1 et 0 ≤ y ≤ 1.

En particulier, dans la suite, on considère le cas particulier où le premier agent photoluminescent est formé par des nanoparticules de Gd_{0,6}Eu_{0,4}VO₄.

Selon une variante préférée, ces nanoparticules sont réduites, préalablement à leur mise en contact avec l'échantillon à analyser.

Les nanoparticules réduites d'un tel premier agent photoluminescent peuvent notamment être obtenues lors d'une étape 14 antérieure consistant à réduire des nanoparticules pour obtenir le premier agent photoluminescent.

Comme détaillé dans la suite du texte, la réduction peut être effectuée :
- par voies physiques, notamment par irradiation laser, électronique ou gamma, par exemple ; ou
- par voie chimique, à l'aide d'un agent chimique réducteur.

Selon une première variante de réalisation, les nanoparticules d'AₓEu₁₋ₓ(VO₄)_{y}(PO₄)_{(1-y)} sont, préalablement à leur mise en contact avec l'échantillon à analyser, réduites par voie physique, en particulier par excitation laser.

Dans le cadre de cette variante de réalisation, les nanoparticules, lorsqu'elles sont mises en contact avec l'échantillon à analyser contiennent déjà de l'europium sous une forme Eu²⁺. Par exemple, dans le cas de la mise en œuvre de particules de Gd_{0,6}Eu_{0,4}VO₄, les ions Eu³⁺ des particules peuvent être réversiblement réduits en ions Eu²⁺ à l'étape 14 du procédé de l'invention.

Il est entendu que cette espèce réduite des nanoparticules d'AₓEu₁₋ₓ(VO₄)_{y}(PO₄)_{(1-y)}, peut également être générée *in situ* c'est-à-dire dans l'échantillon à doser, par exemple par photoréduction de nanoparticules d'AₓEu₁₋ₓ(VO₄)_{y}(PO₄)_{(1-y)} dans lesquelles l'espèce Eu est essentiellement sous forme Eu³⁺. Ce mode de réalisation est notamment décrit dans Casanova et al., Nat Nanotech (2009).

Cependant, la variante de réalisation consistant à mettre en œuvre à titre de réactif de départ des nanoparticules, de Gd_{0,6}Eu_{0,4}VO₄ par exemple, incorporant déjà de l'Eu sous forme réduite est préférée pour son efficacité et sa simplicité. Cette variante permet en outre de ne pas affecter l'intégrité de cellules biologiques si présentes dans l'échantillon à doser.

Selon une autre variante de réalisation, les nanoparticules d'AₓEu₁₋ₓ(VO₄)_{y}(PO₄)_{(1-y)} sont, préalablement à leur mise en contact avec l'échantillon à analyser, réduites par voie chimique, à l'aide d'un agent réducteur.

Cette sous-étape de réduction par voie chimique peut être suivie d'une sous-étape ultérieure, consistant à laver les nanoparticules réduites, pour éliminer l'excédent d'agent réducteur. Par exemple, les particules sont diluées dans une solution contenant un agent réducteur, puis centrifugées, puis dispersées de nouveau dans une solution fraîche sans réducteur (eau pure par exemple), permettant ainsi des lavages successifs.

L'agent réducteur mis en œuvre peut notamment être du NaBH4. Une dernière étape peut consister alors à laver les nanoparticules réduites, pour éliminer l'excédent d'agent réducteur.

Par exemple, les nanoparticules de Gd_{0,6}Eu_{0,4}VO₄ peuvent être réversiblement réduites avec du borohydrure de sodium (NaBH4) à l'étape 14.

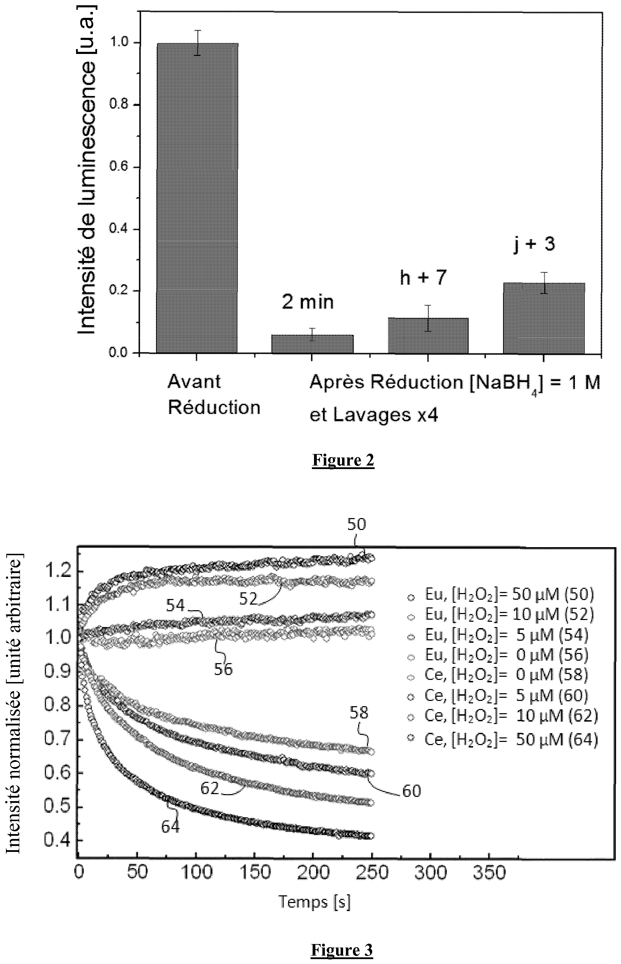

La figure 2 montre la comparaison entre les niveaux de luminescence d'une solution de nanoparticules de Gd_{0,6}Eu_{0,4}VO₄ sous illumination laser de longueur d'onde 466 nm, avant, immédiatement, 7 h et 2 jours après traitement avec 1M de NaBH4.

Les inventeurs ont montré que les particules obtenues suite à la réduction par un agent chimique sont capables de détecter des espèces oxydantes, par exemple H₂O₂, de façon similaire à la détection du H₂O₂ par des particules aux ions Eu³⁺ réduits préalablement en Eu²⁺ suite à une excitation laser.

Ainsi, la demande décrit un procédé utile pour l'analyse d'espèces oxydantes dans un échantillon, en particulier dans un échantillon biologique, comprenant les étapes consistant à :
i) disposer de nanoparticules photoluminescentes d'AₓEu₁₋ₓ(VO₄)_{y}(PO₄)_{(1-y)}, où A est l'un parmi Y, Gd et La, 0 ≤ x ≤ 1 et 0 ≤ y ≤ 1, lesdites nanoparticules étant préalablement réduites, en particulier par voie chimique à l'aide d'un agent réducteur, en particulier par NaBH4 ;
ii) introduire lesdites nanoparticules dans l'échantillon à doser,
iii) exciter lesdites nanoparticules,
iv) mesurer l'intensité lumineuse émise par l'échantillon à au moins une longueur d'onde représentative de la forme oxydée ou de la forme réduite desdites nanoparticules, et
iv) apprécier la présence et/ou quantité d'espèce(s) oxydante(s) par interprétation de ladite mesure, le cas échéant par référence à un étalon ou étalonnage.

Il n'était nullement évident que la réduction des particules d'AₓEu₁₋ₓ(VO₄)_{y}(PO₄)_{(1-y)} par un agent chimique, par exemple NaBH4, puisse permettre de conduire à des nanoparticules oxydables capables de détecter des espèces oxydantes, par exemple H₂O₂.

Sans vouloir être lié par la théorie, les inventeurs ont montré que le mécanisme de réduction n'est pas le même dans les deux cas.

Dans le cas de la réduction par excitation laser, le résultat de l'excitation laser résonante avec les transitions électroniques des ions Eu³⁺ est l'apparition d'ions Eu²⁺, ce qui rend les particules oxydables et capables de détecter l'espèce oxydantes. En revanche, dans le cas de la réduction des nanoparticules par un agent chimique, il n'y pas de modification significative persistante du nombre d'ions Eu³⁺, la réduction par un agent chimique faisant probablement intervenir un phénomène de quenching sur l'émission des ions Eu³⁺, la réoxydation de la particule permettant le retour de luminescence.

### Second agent optiquement actif

Le second agent optiquement actif et de préférence photoluminescent est quant à lui choisi de telle sorte que sa luminescence (ou l'intensité lumineuse de sa luminescence) est constante ou, de manière préférée, varie également avec la quantité d'espèces oxydantes.

Ainsi selon une première variante du procédé de détection d'espèces oxydantes 10 décrit ci-avant, l'intensité de luminescence du deuxième agent optiquement actif, notamment photoluminescent, est sensiblement constante en présence d'espèces oxydantes.

Par sensiblement constante, on entend que la variation d'intensité lumineuse est comprise entre plus ou moins 10 %, de préférence plus ou moins 5 %. Dans ce cas, le second agent optiquement actif peut notamment comprendre ou être constitué par des nanoparticules de LaPO₄:Eu, LaPO₄:Er, LaPO₄:Nd, LaPO₄:Tb, LaF₃:TR (TR=terre rare), NaYF₄:Yb, NaYF₄:Er, YAG:Eu, des nanocristaux semiconducteurs fluorescents CdSe/ZnS, CdTe/ZnS, ou des particules présentant des propriétés d'émission par génération de seconde harmonique telles que KTP et/ou BaTiO₃. Cette variante du procédé permet également de détecter la présence d'espèces oxydantes dans un échantillon testé.

Selon une seconde variante préférée du procédé de détection d'espèces oxydantes 10 décrit ci-avant, l'intensité de luminescence du deuxième agent optiquement actif, varie en fonction de la quantité d'espèces oxydantes, dans un sens opposé à la variation de luminescence observée à ladite première longueur attitrée du premier agent photoluminescent.

Ainsi, dans la suite de la description, on considère que la luminescence du deuxième agent photoluminescent est décroissante avec la quantité d'espèces oxydantes, en présence desquelles il se trouve. Par exemple, le deuxième agent photoluminescent peut consister en une solution contenant des nanoparticules de YAG:Ce et/ou LaPO₄:Ce. Dans la suite de la description, on considère que le deuxième agent photoluminescent est formé par des nanoparticules de YAG:Ce.

La figure 3 illustre les courbes 50-56 et 58-64 de réponse en intensité d'émission des particules de Gd_{0.5}Eu0_{0.4}VO₄ réduites et de YAG:Ce, respectivement, dans un milieu oxydant de concentration connue en peroxyde d'hydrogène, concentration égale à 0 µM, 5 µM, 10 µM et 50 µM, respectivement. Chaque courbe 50-64 est la moyenne de suivis d'environ 10 nanoparticules détectées individuellement.

### Détermination de la présence ou non d'espèces oxydantes selon l'invention

Le procédé de détermination de la présence d'espèces oxydantes 10 se poursuit par une étape 16, consistant à exciter les premier et deuxième agents photoluminescents dans l'échantillon. Pour ce faire, l'échantillon peut être soumis à une double excitation lumineuse, notamment par laser. Dans le cas des exemples des agents photoluminescents considérés dans la présente description, cette double excitation de l'échantillon peut par exemple être réalisée aux longueurs d'onde de 396 nm (pour Gd_{0.6}Eu0_{0.4}VO₄) et 488 nm (pour YAG:Ce), respectivement au moyen d'une diode laser et d'un laser Argon.

Le procédé de détection d'espèces oxydantes 10 se poursuit alors par une étape 18 consistant à mesurer l'intensité lumineuse émise par l'échantillon à au moins une première longueur d'onde correspondant à une longueur d'onde de luminescence du premier agent photoluminescent, et à au moins une deuxième longueur d'onde, correspondant à une longueur d'onde de luminescence du deuxième agent photoluminescent, les première et deuxième longueurs d'onde étant distinctes. Dans l'exemple considéré ici, l'intensité lumineuse émise par l'échantillon est mesurée aux deux longueurs d'onde de luminescence de Eu³⁺ et Ce³⁺, à savoir 617 nm et 550 nm, respectivement.

En effet, la présence d'espèces oxydantes dans l'échantillon provoque, par exemple, l'oxydation des ions Eu²⁺ et Ce³⁺ en ions Eu³⁺ et Ce⁴⁺, respectivement. Ceci se traduit par une baisse de l'intensité lumineuse d'émission des particules à base de cérium à la longueur d'onde de 550 nm et par une augmentation de l'intensité lumineuse des particules à base d'europium à la longueur d'onde de 617 nm.

Dans la variante de réalisation où le deuxième agent possède une luminescence constante quelle que soit la concentration en espèces oxydantes à analyser, son signal lumineux attitré peut être directement utilisé comme échelle de référence. Ainsi, ce signal permet de mettre en évidence soit une modification de l'intensité lumineuse de la ou d'une des longueurs d'onde représentatives de la forme oxydée ou de la forme réduite du premier agent photoluminescent, soit un défaut de variabilité de cette intensité. Une modification de l'intensité lumineuse est représentative de la présence d'espèces oxydantes. Au contraire, un défaut de variabilité de cette intensité lumineuse est révélateur de l'absence d'espèce oxydante au sein de l'échantillon à analyser.

Dans une autre variante de réalisation, le procédé comprend une étape 20 de comparaison des intensités lumineuses mesurées, à l'échelle de la nanoparticule individuelle, de la luminescence de chacun des agents photoluminescents à des valeurs étalon, afin de déterminer une quantité d'espèces oxydantes dans l'échantillon testé. Cette étape est notamment nécessaire dans le cas où l'intensité lumineuse du second agent photoluminescent évolue également avec la quantité d'espèces oxydantes dans l'échantillon à analyser. La quantité d'espèce oxydante peut notamment être une concentration d'espèces oxydantes dans l'échantillon testé.

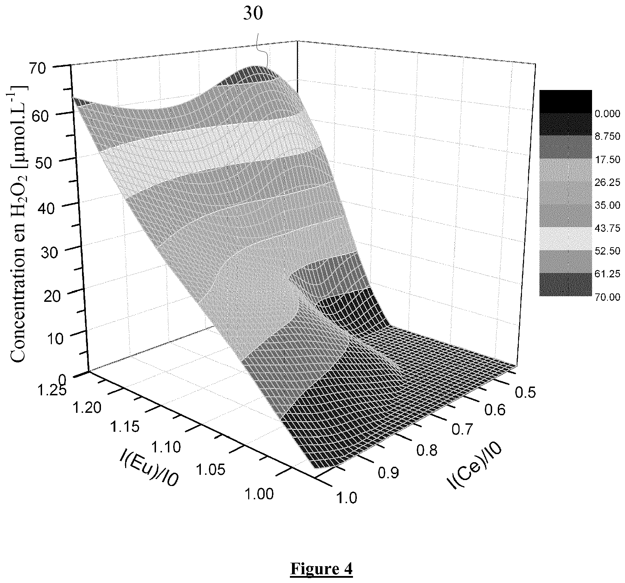

Ici, les valeurs étalon peuvent être déterminées à partir d'une nappe 30 telle qu'illustrée à la figure 4. Cette nappe représente un ensemble de points correspondant à un triplé :
- intensité lumineuse de la luminescence de l'agent photoluminescent comprenant les particules à base d'europium,
- intensité lumineuse de la luminescence de l'agent photoluminescent comprenant les particules à base de cérium, et
- concentration en espèces oxydantes (H₂O₂ dans le cas représenté de la figure 3).

Cette nappe 30 peut notamment être obtenue au moins partiellement à partir des courbes 50-64 de la figure 3. Ici, en effet, les deux agents photoluminescents ayant une réponse opposée en luminescence en fonction de la quantité d'espèces oxydantes, chaque couple de courbes 50-64 correspondant à une même concentration en espèces oxydantes, permet de déterminer un point de la nappe 30. En variante, la nappe 30 peut être interpolée à partir de l'ensemble des données des courbes 50-64 où un couple d'intensités lumineuses normalisées correspond de façon unique à une concentration en espèces oxydantes.

Ainsi, de manière avantageuse, en cas de dépendances monotones et distinctes des luminescences des deux agents avec la concentration d'oxydants, il est possible de construire une nappe déterminant de façon unique une concentration d'oxydant à partir des luminescences instantanées des deux agents. Il est possible d'envisager plusieurs types de réponse en luminescence pour les deux agents. Cependant, une variante préférée est l'utilisation de deux agents ayant des variations de luminescence opposées en réponse aux oxydants.

En variante de cette nappe 30, les valeurs étalons peuvent être issues d'une table ou d'un logiciel de calcul, lequel peut également, à partir de points mesurés, interpoler une concentration en espèces oxydantes, d'un couple d'intensités mesurées.

Dans tous les cas, cette comparaison du couple d'intensités lumineuses mesurées à des valeurs étalons permet de s'affranchir de la mesure de la dérivée des intensités lumineuses, nécessaire pour la méthode décrite dans Casanova et al., Nat. Nanotech (2009). Le procédé présente ainsi une meilleure résolution temporelle.

L'intensité lumineuse mesurée aux longueurs d'onde de luminescence des ions Eu³⁺ et Ce³⁺ permet donc de déterminer un point de la nappe 30, correspondant à une unique concentration d'espèces oxydantes présentes dans l'échantillon testé. L'unicité de cette concentration est assurée par les variations distinctes et monotones de l'intensité de luminescence des agents photoluminescents en fonction de la quantité d'espèces oxydantes avec lesquelles ils sont en présence.

Le procédé de détection d'espèces oxydantes conforme à l'invention est donc particulièrement avantageux pour déterminer rapidement et relativement simplement une quantité d'espèces oxydantes dans un échantillon à partir d'une mesure - double - d'intensité lumineuse.

Ce procédé permet une mesure ratiométrique. Notamment, dans le cas où la luminescence du premier agent photoluminescent varie en fonction de la quantité d'espèce oxydantes, mais que l'intensité lumineuse émise par le deuxième agent optiquement actif est sensiblement constante qu'elle que soit la concentration en espèces oxydantes, il peut être intéressant d'utiliser le rapport entre les intensités d'émission des premiers et deuxième agents pour déterminer la concentration en espèces oxydantes.

Le procédé est en outre particulièrement adapté à une détection d'espèces réactives oxydantes (ou ROS) en volume. En effet, une telle détection ne dépend ni de la concentration d'agents photoluminescents, ni du volume de l'échantillon sondé. Le procédé peut notamment être mis en œuvre pour suivre la production d'espèces oxydantes dans des tissus vivants (prélevés *ex vivo* ou *in vivo*)*.*

Il est rappelé ici que les ROS sont connus pour être produits de façon anormale dans des pathologies telles que certains cancers, maladies neurodégénératives et cardiovasculaires. Le procédé décrit peut donc complémenter les nombreuses études menées dans ce domaine et contribuer ainsi à répondre aux enjeux de santé publique soulevés par ces pathologies.

Le procédé proposé est également avantageusement ajustable à des concentrations plus ou moins importantes d'espèces oxydantes, notamment en modifiant l'intensité lumineuse d'excitation des agents photoluminescents. Les espèces oxydantes pouvant être détectées au moyen du procédé incluent notamment, mais pas exclusivement, H₂O₂, NO et ClO. Le procédé permet de déterminer une concentration absolue d'espèces oxydantes dans l'échantillon. Il présente une meilleure résolution spatiale et temporelle que les procédés connus. Il ne provoque pas de réaction parasite et est biocompatible. Il peut être mis en œuvre pour doser des espèces oxydantes dans des cellules ou des tissus biologiques, mais également pour détecter et/ou doser des espèces oxydantes en solution de molécules biologiques.

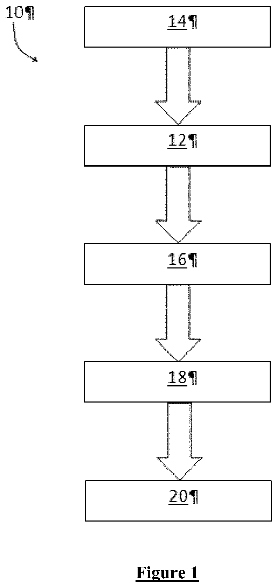

Le procédé 10, décrit ci-avant, peut notamment être mis en œuvre au moyen d'un système 100 de détermination de la présence d'espèces oxydantes tel qu'illustré à la figure 1. Sur cette figure 1, le système de détermination de la présence d'espèces oxydantes dans un échantillon 102 comporte tout d'abord un dispositif d'illumination laser 104 formé ici de deux sources lasers 106, 108 distinctes, adaptées à émettre des faisceaux à des longueurs d'onde distinctes. Dans l'exemple considéré ci-avant où les agents photoluminescents sont du Gd_{0.6}Eu0_{0.4}VO₄ et du YAG:Ce, la première source laser 106 est adaptée à émettre un premier faisceau laser 110 à une longueur d'onde de 488 nm, tandis que la deuxième source laser 18 émet un deuxième faisceau laser 112 de longueur d'onde de 396 nm.

Le système 100 comprend ensuite un miroir dichroïque 114 pour combiner les premier et deuxième faisceaux lasers 110, 112, et les guider vers un objectif optique 116 adapté à focaliser les faisceaux lasers 110, 112 sur une zone de l'échantillon 102 à tester. Cette zone peut par exemple correspondre à une surface de quelques mm². Il est à noter ici que l'échantillon 102 peut être disposé sur ou dans un support d'échantillon 103. Notamment, dans le cas où l'échantillon est gazeux, ce support d'échantillon 103 prend la forme d'un volume fermé pour empêcher la dispersion de l'échantillon 102 à tester. Le support d'échantillon 103 peut également prendre la forme d'une cuve ou cuvette, en particulier dans le cas où l'échantillon 102 est sous forme de solution.

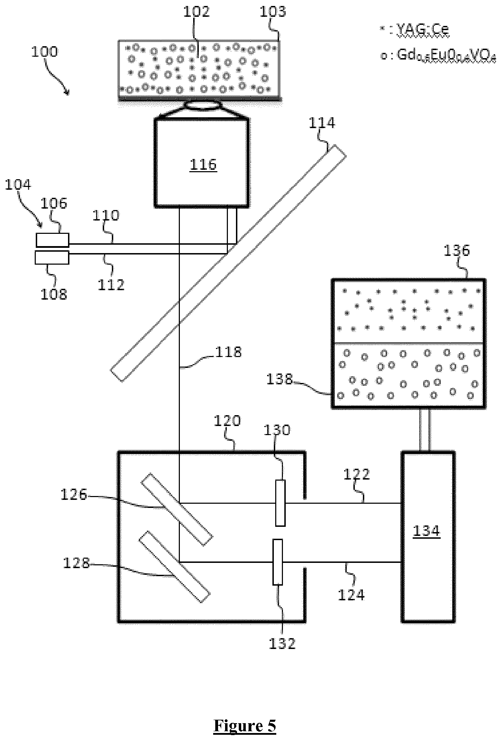

L'échantillon à tester est mis en contact avec le premier agent photoluminescent et le deuxième agent optiquement actif, ici photoluminescent. Comme dans l'exemple considéré ci-avant, les agents photoluminescents prennent ici la forme de deux types de particules différentes :
- des particules de YAG:Ce (notées « * » dans la figure 5), et
- des particules réduites de Gd_{0.6}EuO_{0.4}VO₄, (notées « o » dans la figure 5).

Les premier et deuxième faisceaux lasers 110, 112, excitent les premier et deuxième agents photoluminescents dans l'échantillon 102. Les agents photoluminescents, de types différents, répondent à l'excitation d'un des deux faisceaux laser, de préférence de manière sélective (c'est-à-dire exclusivement à l'un ou l'autre), en émettant dans deux gammes de longueurs d'ondes différentes. Par « deux gammes de longueurs d'ondes différentes », on entend deux gammes de longueurs d'ondes comprenant au moins une longueur d'onde non comprise dans l'autre gamme de longueurs d'onde. Par exemple, ici, les particules de YAG:Ce répondent à l'excitation du premier faisceau laser à 488 nm, en émettant à une première longueur d'onde de 550 nm. Les particules réduites de Gd_{0.6}EuO_{0.4}VO₄, elles, répondent à l'excitation du deuxième faisceau laser à 396 nm en émettant à une deuxième longueur d'onde de 617 nm.

L'objectif 116 permet de focaliser l'émission des deux agents luminescents 118 incluant les deux longueurs d'onde de réponse des agents photoluminescents vers un dédoubleur spectral 120, éventuellement via le miroir dichroïque 114 qu'il traverse. Le filtre dichroïque 114 joue alors le rôle d'une lame séparatrice fonctionnellement interposée entre les sources lasers 106, 108 et l'échantillon 102, d'une part, et entre l'échantillon 102 et le dédoubleur spectral 120, d'autre part, en réfléchissant les sources laser et en étant transparent à l'émission des agents luminescents.

En variante, l'objectif est intégré dans un dispositif optique, notamment un microscope, fonctionnellement interposé entre l'échantillon 102 et le dédoubleur spectral 120. Ce microscope peut notamment être un microscope à fluorescence, de préférence à champ large, un macroscope ou un stéréomicroscope.

Le dédoubleur spectral 120 permet d'obtenir, à partir d'un unique faisceau d'entrée 118, deux images de sortie 122, 124 filtrées à deux longueurs d'ondes différentes, ici les longueurs d'onde de réponse des agents photoluminescents en contact avec l'échantillon 102. En d'autres termes, le dédoubleur spectral 120 permet de filtrer les émissions des agents photoluminescents selon des longueurs d'ondes distinctes et ainsi former deux images filtrées distinctes de l'échantillon 102 à tester.

Pour ce faire, le dédoubleur spectral 120 comporte ici un miroir dichroïque 126. Des premier 130 et deuxième 132 filtres de longueur d'onde respective correspondant aux longueurs d'onde de réponse des agents photoluminescents sont placés respectivement sur le chemin du faisceau 122 réfléchi par le miroir dichroïque 126 et sur le chemin du faisceau 124 traversant le miroir dichroïque 126. Ceci permet de séparer spatialement les faisceaux de longueurs d'onde différentes pour détection sur deux capteurs distincts ou sur deux régions distinctes d'un capteur unique, comme une caméra 134.

Le système 100 comprend encore des moyens de détermination de l'intensité lumineuse associée aux deux images filtrées. Ici, ces moyens comprennent une caméra 134, par exemple une caméra EMCCD (pour « Electron Multiplying Charge Coupled Device ») reliée à un enregistreur, par exemple un ordinateur, pour détecter les deux images filtrées, chacune sur une région respective 136, 138, correspondant à la réponse de chacun des deux agents photoluminescents.

Alternativement, les moyens de détermination de l'intensité lumineuse mis en œuvre comportent deux photo-détecteurs à des longueurs d'ondes distinctes, notamment du type photomultiplicateurs ou photodiodes. Ces photo-détecteurs permettent de mesurer l'intensité lumineuse totale provenant de l'ensemble de l'échantillon éclairé sans résolution spatiale.

Il est à noter ici que le système 100 permet de détecter simultanément et individuellement chaque type de particules. Dans le cas d'utilisation d'un dispositif plein champ, comme la camera 134, les particules peuvent en outre être détectées individuellement et localiser spatialement, du fait de la faible densité surfacique de l'échantillon. Ainsi, le suivi à l'échelle de chaque particule unique de ces deux émissions de luminescence et de leurs évolutions permet une mesure quantitative, locale (notamment avec une précision de localisation de 40 nm) et résolue en temps, avec une cadence d'acquisition de l'ordre de 100 ms, notamment de 33 ms, des espèces oxydantes dans l'échantillon. La résolution temporelle de la mesure est en effet alors donnée par la cadence d'acquisition de la caméra 134.

Les puissances des lasers d'excitation peuvent par ailleurs être modulées pour changer la gamme de concentrations d'espèces oxydantes détectées. Le procédé et le système de détection d'espèces oxydantes peuvent ainsi notamment être mis en œuvre dans de nombreuses applications, comme la détection des ROS dans des inflammations ou certaines tumeurs.

### EXEMPLES

### Exemple 1

Ci-après, on décrit plus précisément un exemple d'expérience menée pour détecter des espèces oxydantes produites dans une cellule mammifère suite à une stimulation par une protéine.

Dans un premier temps, des cellules de mammifères ont été préparées. Pour ce faire, des cellules musculaires lisses vasculaires de souris (culture primaire) ont été cultivées dans du RPMI (pour « Roswell Park Mémorial Institute » médium) contenant 10 % de sérum fœtal de veau et des antibiotiques, en l'espèce de la pénicilline et de la streptomycine. Lorsque la confluence atteint 80 %, les cellules sont prélevées et déposées sur des lamelles de verre. Dans le cas d'espèce, 1,5x10⁴ cellules sont typiquement disposées sur une lamelle de verre. Les cellules sont ensuite cultivées sur les lamelles pendant quarante-huit heures dans le milieu de culture complet, puis dans un milieu sans sérum pendant vingt-quatre heures.

Par ailleurs, une solution colloïdale stable de Gd_{0,4}EuO_{0,6}VO₄ de concentration sensiblement égale à 1 mM est préparée. La solution est ensuite centrifugée pendant cinq minutes sous une accélération de 5000 g afin de faire précipiter les particules. On prélève le surnageant et on suspend à nouveau le culot dans une solution aqueuse de borohydrate de sodium (NaBH4) de concentration 1 M. La réaction du NaBH4 avec les particules et avec l'eau produit du dihydrogène sous forme de bulles. On veille donc à ne pas confiner le milieu réactionnel. Après quelques minutes, la solution est de nouveau centrifugée, cinq minutes, sous une accélération de 5000 g. Le surnageant est prélevé et le culot est suspendu de nouveau dans l'eau distillée. On renouvelle l'opération de lavage deux fois afin d'éliminer d'éventuels ions réducteurs résiduels.

Une solution stable de YAG:Ce (4 %) d'environ 1 mM est préparée, ayant pour solvant principal l'eau. Ces particules sont synthétisées par voie glycothermale sous forme de dispersions dans l'éthanol. Des cycles de lavages sont réalisés : les centrifugations nécessaires peuvent être effectuées pendant 5 minutes sous une accélération de 5000 g puis les particules sont de nouveau suspendues dans le solvant choisi pour les mesures, l'eau dans le cas décrit ici.

On mélange par ailleurs dans une solution commerciale de milieu hypertonique (LifeTechnologies), 10 µL de suspension de YAG:Ce et 15 µL de suspension de particules de Gd_{0,4}EuO_{0,6}VO₄ réduites et lavées. On incube les cellules avec cette solution pendant dix minutes puis deux minutes dans une solution hypotonique contenant 70 % de RPMI et 30 % d'eau distillée stérile. On incube ensuite les cellules dans leur milieu complet pendant trente à soixante minutes.

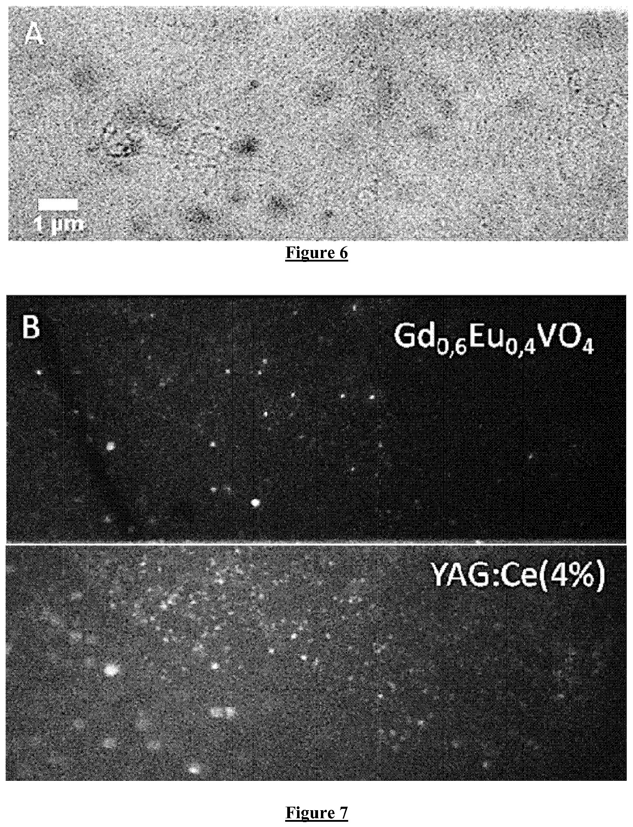

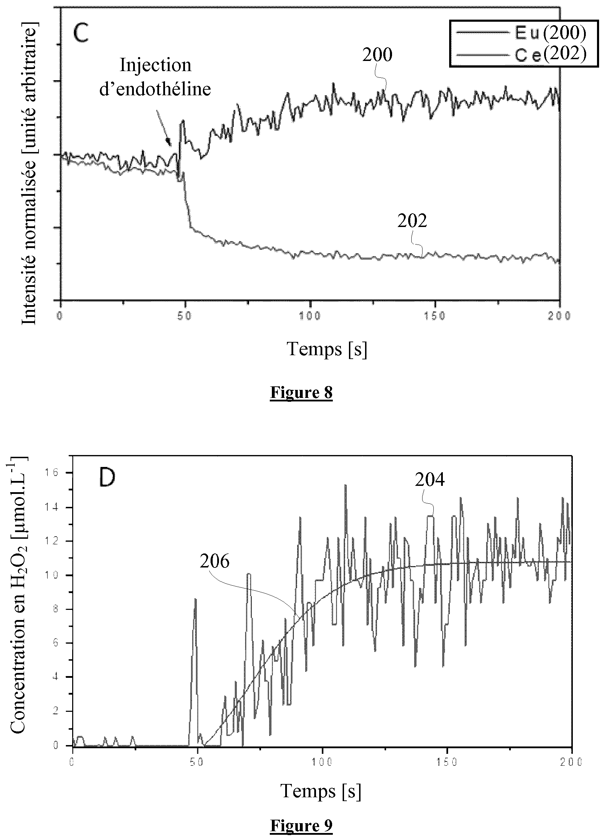

On dépose alors une lamelle contant des cellules chargées de nanoparticules sur un porte-échantillon et on ajoute 1 mL de milieu physiologique tamponné (HBSS/1 mM HEPES). On monte alors la lamelle sur un microscope à épifluorescence pour l'observation de particules individuelles (Olympus IX71, Objectif x63 NA=1.4). On observe alors les cellules en transmission de lumière blanche, comme illustré à la figure 6, et sous illumination duale (sources laser à 488 nm et 396 nm), comme illustré à la figure 7. On choisit une cellule présentant une morphologie adéquate, c'est-à-dire une cellule vivante et adhérente. Après cinquante secondes d'observation, on remplace le milieu initial par un milieu physiologique contant 270 nM d'endothéline-1 (ET-1) et on observe la luminescence de nanoparticules individuelles pendant plusieurs minutes sur un capteur plein champ (caméra EM-CCD ImageEM, Hamamatsu). La figure 8 illustre les courbes réponses 200, 202 correspondant pour les particules à base d'Eu et de Ce, respectivement. On observe une augmentation de l'intensité lumineuse des particules à base d'Eu et une diminution de l'intensité lumineuse des particules à base de Ce.

Par la suite, on détermine les variations d'intensité des particules observées (dopées au Ce ou à l'Eu) au cours du temps grâce à un logiciel de traitement d'images ou de calcul (par exemple Matlab). On normalise chaque courbe obtenue en prenant pour référence les premières valeurs mesurées. On extrait les concentrations de peroxyde d'hydrogène grâce à la nappe 30 de la figure 4. On obtient ainsi la courbe 204 illustrée à la figure 9, et la courbe lissée 206 correspondante, qui donne la variation de la concentration en H₂O₂ dans l'échantillon testé, en fonction du temps.

### Exemple 2

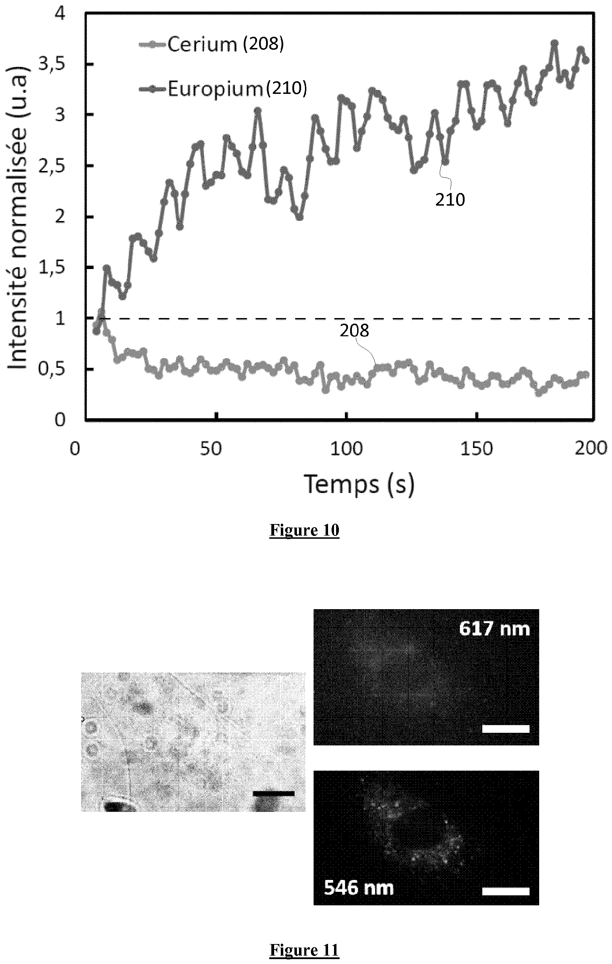

La figure 10 illustre un autre exemple d'utilisation du procédé selon l'invention, dans lequel l'échantillon à analyser est contenu dans une cuve. Les deux agents, respectivement YAG:Ce et GdVO₄:Eu sont introduits dans la cuve en solution. Ils sont excités par des sources spécifiques. Après ajout de 2 mM de peroxyde d'hydrogène à t=0), la luminescence de chacun des agents, détectée à 550 nm et 617 nm, est collectée sur deux photomultiplicateurs, pour former les deux courbes 208, 210, respectivement.

### Exemple 3

Les cellules musculaires lisses vasculaires tapissent la paroi des vaisseaux sanguins. Leur stimulation par un vasoconstricteur puissant tel que l'endothéline-1 (ET-1) produit leur contraction et induit l'augmentation de la pression artérielle. Le processus de signalisation cellulaire responsable d'une telle réponse est connu pour induire une production de peroxyde d'hydrogène (espèce oxydante). Par ailleurs, les récepteurs membranaires EGFR (*Epidermal Growth Factor Receptor*) sont également connus pour leur participation à ce processus. En revanche, leur contribution et en particulier leur effet sur la production d'oxydants sont inconnus à ce jour. Ceci est dû à l'inexistence de procédés de détection quantitatifs et assez rapides pour caractériser ce phénomène.

Dans cet exemple, il a été étudié la transactivation des récepteurs EGFR dans la réponse au vasoconstricteur ET-1 par le procédé de l'invention.

Pour cela, le même protocole que l'Exemple 1 a été suivi, à savoir la culture de cellules musculaires lisses vasculaires, la préparation de la suspension de particules YAG:Ce et Gd_{0,4}EuO_{0,6}VO₄ réduites, et l'incubation des cellules cultivées avec la suspension.

La présence de ces particules a été observée par microscopie de fluorescence et dédoublement spectral, comme indiqué dans l'Exemple 1 (Figure 11). Un mélange de nanoparticules (GdVO₄ :Eu pré-réduites chimiquement et YAG :Ce) est internalisé dans des cellules musculaires lisses vasculaires déposées sur une lamelle de verre (170 µm) montée lamelle sur un microscope à épifluorescence pour l'observation de particules individuelles (Olympus IX71, Objectif x63 NA=1.4) muni d'un dédoubleur spectral permettant de projeter sur deux régions distinctes d'une caméra ultra-sensible (EM-CCD, Evolve 512 Roper Scientific) la photoluminescence de chaque type de particule.

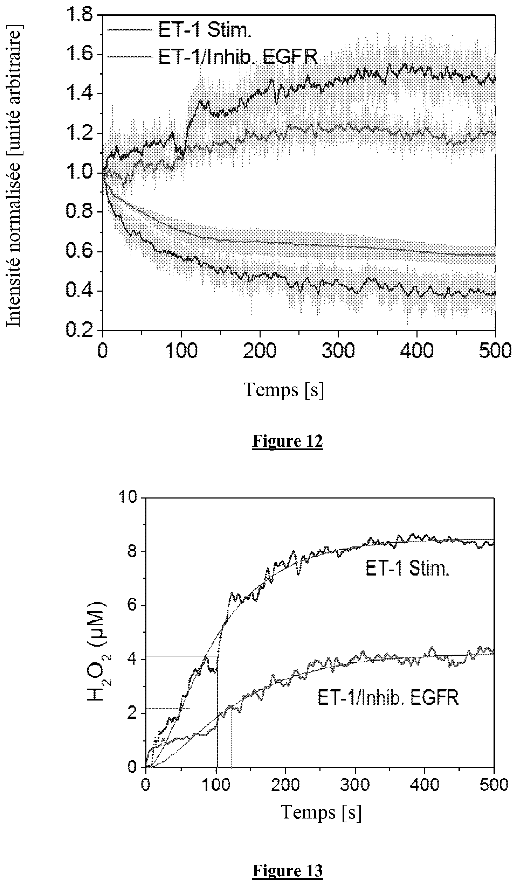

La stimulation de cellules normales et de cellules où les EGFR ont spécifiquement été inhibés par la présence de l'agent AG1478 (application de 30 min avant stimulation et ajout dans le milieu d'observation sous microscope) a été réalisée avec une concentration de 270 nM d'endothéline-1. Immédiatement après stimulation, les signaux de luminescence émis par les différentes particules dans les cellules ont été collectés. En figure 12 sont ainsi représentées les photoluminescences normalisées par leur valeur initiale des nanoparticules YAG :Ce (au-dessous de 1) et GdVO₄ :Eu (au-dessus de 1) obtenues après stimulation par ET-1 (avec ou sans inhibition des EGFRs).

Par la suite, grâce à une calibration décrite ci-dessus, l'évolution temporelle des concentrations absolues de peroxyde d'hydrogène a été déduite (Figure 13) dans les deux conditions cellulaires (normales ou EGFR inhibés). Cette étape permet de quantifier la contribution de la voie EGFR au processus global de signalisation. Cette contribution s'élève à 50% puisque les cellules inhibées produisent deux fois moins de peroxyde d'hydrogène que les cellules normales. Elle permet de plus de quantifier l'effet de cette inhibition à la cinétique de production de peroxyde d'hydrogène, au contraire par exemple de Bouzigues et al., Chem. Biol., 2014 ; 21 :647-56, en révélant que la transactivation des EGRs se produit en moins d'1 seconde et contrôle cette cinétique de production aux temps < 1 min.

Bien entendu la présente invention ne se limite pas aux seuls exemples décrits ci-avant mais est susceptibles de nombreuses variantes accessibles à l'homme de l'art, dans le cadre des revendications ci-jointes.

Ainsi, par exemple, le deuxième agent optiquement actif peut être constitué de tout type d'agent photostable, comme des nanocristaux semi-conducteurs, par exemple.

Par ailleurs, les particules peuvent être fonctionnalisées et couplées à une ou plusieurs biomolécules d'intérêt notamment à des fins de cibler soit des compartiments cellulaires spécifiques dans le cas de mesures au niveau cellulaire, soit des types cellulaires spécifiques par exemple des cellules cancéreuses, dans le cas de mesures au niveau des tissus ou *in vivo* (dans des organismes vivants).

Plus précisément, les molécules biologiquement actives sont des molécules de ciblage, c'est-à-dire des molécules qui vont permettre le ciblage spécifique de la particule selon l'invention vers un organe, un fluide corporel (par exemple le sang), un type cellulaire (par exemple, plaquettes, lymphocytes, monocytes, cellules tumorales, ...) ou un compartiment cellulaire. Ainsi, ce ciblage spécifique peut être accompli à l'aide d'anticorps, monoclonal ou polyclonal, ou de ligands protéiques ou polypeptidiques de récepteurs cellulaires. À titre d'exemples, non limitatifs, on peut citer les couples récepteurs/ligands suivants : TGF/TGFR, EGF/EGFR, TNFa/TNFR, interféron/récepteur à l'interféron, interleukine/récepteur à l'interleukine, GMCSF/récepteur au GMCSF, MSCF/récepteur au MSCF, et GCSF/récepteur au GCSF. On peut également citer, comme ligands, des fragments de toxine ou des toxines détoxifiées et leurs récepteurs cellulaires. Concernant les anticorps, ceux-ci seront choisis en fonction de l'antigène ou des antigènes contre lequel/lesquels l'anticorps est dirigé.

Dans un autre mode de réalisation, la ou les molécule(s) biologiquement active(s) sont des agents de furtivité, tels que le polyéthylèneglycol (PEG) ou le dextran, afin de rendre les particules furtives dans l'organisme et ainsi augmenter leur temps de circulation dans le sang.

Dans un mode de réalisation particulier, les particules selon l'invention peuvent être fonctionnalisées par des molécules de ciblage et des molécules de furtivité telles que définies ci-dessus.

Quel que soit le mode de réalisation, les molécules biologiquement actives peuvent être attachées, à la surface de la particule ou le cas échéant à la couche de préparation, directement ou par l'intermédiaire d'une couche portant des groupements fonctionnels, par liaison covalente ou non-covalente. L'attachement de ces molécules biologiquement actives est réalisé par les techniques conventionnelles d'oxydation, halogénation, alkylation, acylation, addition, substitution ou amidation de la surface de la particule, de la couche de préparation et/ou de la couche portant des groupements fonctionnels, avec les molécules biologiquement actives.

La couche de préparation est appliquée directement sur la particule, soit par liaison covalente ou par absorption. Cette couche de préparation peut être hydrophile ou hydrophobe. Dans un mode de réalisation particulier, cette couche de préparation est amorphe.

Le couplage entre les particules et une biomolécule est par exemple décrit dans la demande WO-A-2012/010811. La demande WO-A-2013/123197 décrit en outre l'enrobage des particules pouvant mener à des particules couplée à des biomolécules.

Par ailleurs, afin d'améliorer le rapport signal à bruit, il est possible d'utiliser une détection retardée du signal émis par les particules dopées europium, dont la durée de vie d'état excité est longue comparée à la plupart des fluorophores. A l'aide d'un dispositif d'occultation, notamment mécanique ou électronique, des moyens de détermination de l'intensité lumineuse, sélectivement activable, cette approche consiste à ne détecter, après une impulsion laser d'excitation des premier et deuxième agents, que les photons émis après un temps déterminé. Ce temps déterminé peut être choisi afin de ne pas détecter les photons provenant d'autres émetteurs, par exemple par autofluorescence, et à détecter uniquement des photons émis par les nanoparticules YVO₄:Eu.

## Revendications

1. Procédé utile pour l'analyse d'espèces oxydantes dans un échantillon, en particulier dans un échantillon biologique, comprenant les étapes consistant à :
i) mettre en contact (12) l'échantillon avec un premier agent photoluminescent, et un deuxième agent optiquement actif, au moins le premier agent photoluminescent comportant des nanoparticules dopées aux terres rares et oxydables, avec
- la luminescence du premier agent photoluminescent variant, à au moins une première longueur d'onde, avec la quantité d'espèces oxydantes, et
- le signal émis par le deuxième agent optiquement actif étant, à au moins une seconde longueur d'onde distincte de la première longueur d'onde, constant avec la quantité d'espèces oxydantes, ou
variant avec la quantité d'espèces oxydantes dans un sens opposé à la luminescence du premier agent photoluminescent,
ii) exciter (16) le premier agent photoluminescent et le deuxième agent optiquement actif dans l'échantillon
iii) mesurer (18) l'intensité lumineuse de l'échantillon à au moins ladite première longueur d'onde, et au moins ladite seconde longueur d'onde, et
iv) apprécier la présence et/ou la quantité d'espèce(s) oxydante(s) par interprétation (20) desdites intensités lumineuses mesurées, et le cas échéant par référence à des valeurs étalon.

2. Procédé selon la revendication 1 dans lequel, à l'étape iv), une quantité d'espèces oxydantes dans l'échantillon est déterminée par lecture (20) sur une nappe étalon (30) de la valeur correspondant au couple d'intensités lumineuses mesurées à l'étape iii), la nappe étalon étant plus particulièrement une nappe (30) préétablie au moyen de mesures réalisées avec des échantillons à quantité connue en espèces oxydantes.

3. Procédé selon l'une quelconque des revendications 1 et 2, dans lequel l'intensité lumineuse de la luminescence du premier agent photoluminescent est croissante avec la quantité d'espèces oxydantes.

4. Procédé selon l'une quelconque des revendications 1 à 3, dans lequel l'intensité lumineuse d'émission du deuxième agent optiquement actif est sensiblement constante avec la quantité d'espèces oxydantes.

5. Procédé selon l'une quelconque des revendications 1 à 4, dans lequel le deuxième agent optiquement actif est photoluminescent et comporte, de préférence, des nanoparticules de LaPO₄:Eu, LaPO₄:Er, LaPO₄:Nd, LaPO₄:Tb, LaF₃:TR, NaYF₄:Yb, NaYF₄:Er, CdSe/Zns, CdTe/ZnS, KTP et/ou BaTiO₃.

6. Procédé selon l'une quelconque des revendications 1 à 3, dans lequel l'intensité lumineuse d'émission du deuxième agent optiquement actif est décroissante avec la quantité d'espèces oxydantes.

7. Procédé selon l'une quelconque des revendications 1 à 3 et 6, dans lequel la première longueur d'onde est représentative de la forme oxydée du premier agent photoluminescent et la deuxième longueur d'onde est représentative de la forme réduite de l'agent optiquement actif et de préférence photoluminescent.

8. Procédé selon l'une quelconque des revendications 1 à 3 et 6 ou 7, dans lequel le deuxième agent optiquement actif est photoluminescent et comporte, de préférence, des nanoparticules de YAG:Ce, LaPO₄:Ce.

9. Procédé selon l'une quelconque des revendications précédentes, dans lequel le premier agent photoluminescent comporte des nanoparticules au moins partiellement réduites d'AₓEu₁₋ₓ(VO₄)_{y}(PO₄)_{(1-y)}, en particulier d'AₓEu₁₋ₓVO₄, où A est l'un parmi Y, Gd et La, 0 ≤ x ≤ 1 et 0 ≤ y ≤ 1.

10. Procédé selon la revendication précédente, comprenant une étape a), antérieure à l'étape i), consistant à réduire (14) des nanoparticules pour obtenir le premier agent photoluminescent, la réduction étant en particulier réalisée par voie chimique, à l'aide d'un agent réducteur, l'étape a) comprenant de préférence une sous-étape, ultérieure à la réduction par voie chimique, consistant à laver les nanoparticules réduites, pour éliminer l'excédent d'agent réducteur, l'agent réducteur contenant plus particulièrement du NaBH4.

11. Procédé selon l'une quelconque des revendications précédentes dans lequel les particules sont en outre, couplées à une ou plusieurs biomolécules fonctionnalisées et/ou biomolécules d'intérêt notoire choisies parmi des molécules de ciblage et des agents de furtivité.

12. Utilisation ex vivo ou in vitro d'un procédé selon l'une quelconque des revendications 1 à 11 à des fins de diagnostic notamment de troubles physiologiques, pathologiques ou non, et associés à une expression non physiologiquement acceptable d'une ou plusieurs espèces oxydantes réactives (ROS).

13. Utilisation ex vivo ou in vitro d'un procédé selon l'une quelconque des revendications 1 à 11 à des fins de criblage de l'efficacité d'un actif à l'égard d'un trouble, pathologique ou non, et associé à une surexpression d'une ou plusieurs espèces oxydantes réactives (ROS).

## Patentansprüche

1. Verfahren zur Analyse von oxidierenden Spezies in einer Probe, insbesondere in einer biologischen Probe, umfassend die in Folgendem bestehenden Schritte:
i) Inkontaktbringen (12) der Probe mit einem photolumineszenten ersten Mittel und einem optisch aktiven zweiten Mittel, wobei mindestens das photolumineszente erste Mittel Nanopartikel beinhaltet, die mit seltenen Erden dotiert und oxidierbar sind, wobei
- die Lumineszenz des photolumineszenten ersten Mittels bei mindestens einer ersten Wellenlänge mit der Menge von oxidierenden Spezies variiert und
- das von dem optisch aktiven zweiten Mittel emittierte Signal bei mindestens einer zweiten Wellenlänge, die von der ersten Wellenlänge verschieden ist,
mit der Menge von oxidierenden Spezies konstant ist oder
mit der Menge von oxidierenden Spezies in eine zur Lumineszenz des photolumineszenten ersten Mittels entgegengesetzte Richtung variiert,
ii) Anregen (16) des photolumineszenten ersten Mittels und des optisch aktiven zweiten Mittels in der Probe,
iii) Messen (18) der Lichtintensität der Probe bei mindestens der ersten Wellenlänge und mindestens der zweiten Wellenlänge und
iv) Beurteilen des Vorkommens und/oder der Menge von einer oder mehreren oxidierenden Spezies durch Interpretieren (20) der gemessenen Lichtintensitäten und gegebenenfalls in Bezug auf Eichwerte.

2. Verfahren nach Anspruch 1, bei dem im Schritt iv) eine Menge von oxidierenden Spezies in der Probe durch Ablesen (20) des Werts, der dem im Schritt iii) gemessenen Lichtintensitätenpaar entspricht, auf einer Eichfläche (30) bestimmt wird, wobei die Eichfläche insbesondere eine Fläche (30) ist, die zuvor mittels Messungen erstellt wurde, die mit Proben mit einer bekannten Menge von oxidierenden Spezies ausgeführt wurden.

3. Verfahren nach einem der Ansprüche 1 und 2, bei dem die Lichtintensität der Lumineszenz des photolumineszenten ersten Mittels mit der Menge von oxidierenden Spezies zunimmt.

4. Verfahren nach einem der Ansprüche 1 bis 3, bei dem die Emissionslichtintensität des optisch aktiven zweiten Mittels mit der Menge von oxidierenden Spezies im Wesentlichen konstant ist.

5. Verfahren nach einem der Ansprüche 1 bis 4, bei dem das optisch aktive zweite Mittel photolumineszent ist und bevorzugt Nanopartikel von LaPO₄:Eu, LaPO₄:Er, LaPO₄:Nd, LaPO₄:Tb, LaF₃:TR, NaYF₄:Yb, NaYF₄:Er, CdSe/ZnS, CdTe/ZnS, KTP und/oder BaTiO₃ beinhaltet.

6. Verfahren nach einem der Ansprüche 1 bis 3, bei dem die Emissionslichtintensität des optisch aktiven zweiten Mittels mit der Menge von oxidierenden Spezies abnimmt.

7. Verfahren nach einem der Ansprüche 1 bis 3 und 6, bei dem die erste Wellenlänge für die oxidierte Form des photolumineszenten ersten Mittels repräsentativ ist und die zweite Wellenlänge für die reduzierte Form des optisch aktiven und bevorzugt photolumineszenten Mittels repräsentativ ist.

8. Verfahren nach einem der Ansprüche 1 bis 3 und 6 oder 7, bei dem das optisch aktive zweite Mittel photolumineszent ist und bevorzugt Nanopartikel von YAG:Ce, LaPO₄:Ce beinhaltet.

9. Verfahren nach einem der vorhergehenden Ansprüche, bei dem das photolumineszente erste Mittel mindestens teilweise reduzierte Nanopartikel von AₓEu₁₋ₓ(VO₄)_{y}(PO₄)_{(1-y)}, insbesondere von AₓEu₁₋ₓVO₄, beinhaltet, wobei A eines unter Y, Gd und La, 0 ≤ x ≤ 1 und 0 ≤ y ≤ 1 ist.

10. Verfahren nach dem vorhergehenden Anspruch, umfassend vor dem Schritt i) einen Schritt a), der darin besteht, Nanopartikel zu reduzieren (14), um das photolumineszente erste Mittel zu erhalten, wobei die Reduktion insbesondere auf chemischem Weg mithilfe eines reduzierenden Mittels ausgeführt wird, wobei der Schritt a) bevorzugt nach der Reduktion auf chemischem Weg einen Teilschritt umfasst, der darin besteht, die reduzierten Nanopartikel zu waschen, um den Überschuss von reduzierendem Mittel zu beseitigen, wobei das reduzierende Mittel insbesondere NaBH₄ beinhaltet.

11. Verfahren nach einem der vorhergehenden Ansprüche, bei dem die Partikel ferner an ein oder mehrere funktionalisierte Biomoleküle und/oder Biomoleküle von allgemein bekanntem Interesse gekoppelt sind, die unter Zielmolekülen und Tarnmitteln gewählt sind.

12. Ex-vivo- oder In-vitro-Verwendung eines Verfahrens nach einem der Ansprüche 1 bis 11 zu Zwecken der Diagnose, insbesondere von physiologischen Störungen, die pathologisch sind oder nicht und die mit einer nicht physiologisch akzeptablen Expression von einer oder mehreren reaktiven oxidierenden Spezies (ROS) assoziiert sind.

13. Ex-vivo- oder In-vitro-Verwendung eines Verfahrens nach einem der Ansprüche 1 bis 11 zu Zwecken des Screenings der Wirksamkeit eines Wirkstoffs bei einer Störung, die pathologisch ist oder nicht und die mit einer Überexpression von einer oder mehreren reaktiven oxidierenden Spezies (ROS) assoziiert ist.

## Claims

1. Method that can be used for analyzing oxidizing species in a sample, in particular in a biological sample, comprising the steps consisting of:
i) contacting (12) the sample with a photoluminescent first agent and an optically active second agent, the photoluminescent first agent at least comprising oxidizable nanoparticles doped with rare earths, with
- the luminescence of the photoluminescent first agent varying, at at least one first wavelength, with the quantity of oxidizing species, and
- the signal emitted by the optically active second agent being, at at least one second wavelength different from the first wavelength,
constant with the quantity of oxidizing species, or
varying with the quantity of oxidizing species in a direction opposite to the luminescence of the photoluminescent first agent,
ii) exciting (16) the photoluminescent first agent and the optically active second agent in the sample
iii) measuring (18) the luminous intensity of the sample at at least said first wavelength, and at least said second wavelength, and
iv) estimating the presence and/or the quantity of oxidizing species by interpretation (20) of said measured luminous intensities, and if applicable by reference to standard values.

2. Method according to Claim 1, in which, in step iv), a quantity of oxidizing species in the sample is determined by reading (20), on a standard sheet (30), the value corresponding to the pair of luminous intensities measured in step iii), the standard sheet more particularly being a sheet (30) established beforehand by means of measurements performed with samples with a known quantity of oxidizing species.

3. Method according to either one of Claims 1 and 2, in which the luminous intensity of the luminescence of the photoluminescent first agent increases with the quantity of oxidizing species.

4. Method according to any one of Claims 1 to 3, in which the luminous intensity of emission of the optically active second agent is roughly constant with the quantity of oxidizing species.

5. Method according to any one of Claims 1 to 4, in which the optically active second agent is photoluminescent and preferably comprises nanoparticles of LaPO₄:Eu, LaPO₄:Er, LaPO₄:Nd, LaPO₄:Tb, LaF₃:RE, NaYF₄:Yb, NaYF₄:Er, CdSe/ZnS, CdTe/ZnS, KTP and/or BaTiO₃.

6. Method according to any one of Claims 1 to 3, in which the luminous intensity of emission of the optically active second agent decreases with the quantity of oxidizing species.

7. Method according to any one of Claims 1 to 3 and 6, in which the first wavelength is representative of the oxidized form of the photoluminescent first agent and the second wavelength is representative of the reduced form of the optically active and preferably photoluminescent agent.

8. Method according to any one of Claims 1 to 3 and 6 or 7, in which the optically active second agent is photoluminescent and preferably comprises nanoparticles of YAG:Ce, LaPO₄:Ce.

9. Method according to any one of the preceding claims, in which the photoluminescent first agent comprises at least partially reduced nanoparticles of AₓEu₁₋ₓ(VO₄)_{y}(PO₄)_{(1-y)}, in particular of AₓEu₁₋ₓVO₄, where A is one from Y, Gd and La, 0 ≤ x ≤ 1 and 0 ≤ y ≤ 1.

10. Method according to the preceding claim, comprising a step a), prior to step i), consisting of reducing (14) nanoparticles to obtain the photoluminescent first agent, the reduction in particular being performed chemically, using a reducing agent, step a) preferably comprising a substep, subsequent to the chemical reduction, consisting of washing the reduced nanoparticles to remove the excess reducing agent, the reducing agent more particularly containing NaBH₄.

11. Method according to any one of the preceding claims in which the particles are additionally coupled to one or more functionalized biomolecules and/or biomolecules known to be of interest selected from targeting molecules and stealth agents.

12. Ex vivo or in vitro use of a method according to any one of Claims 1 to 11 for purposes of diagnosis, notably of physiological disorders, whether or not pathological, and associated with expression of one or more reactive oxygen species (ROS) that is not physiologically acceptable.

13. Ex vivo or in vitro use of a method according to any one of Claims 1 to 11 for purposes of screening the efficacy of an active substance with respect to a disorder, whether or not pathological, and associated with overexpression of one or more reactive oxygen species (ROS).
